# EUROPÄISCHE PATENTSCHRIFT

(11) **EP 0 409 936 B1**
(45) Veröffentlichungstag und Bekanntmachung des Hinweises auf die Patenterteilung: **06.04.1994**
(21) Anmeldenummer: 90901552.1
(22) Anmeldetag: 16.01.1990
(51) Int. Cl.: A61K 9/107, A61K 47/28

(54) **VERFAHREN ZUR HERSTELLUNG WÄSSRIGER MISCHMICELLÖSUNGEN**
PROCESS FOR PRODUCING AQUEOUS MIXED MICELLE SOLUTIONS
PROCEDE DE PRODUCTION DE SOLUTIONS MICELLAIRES MIXTES AQUEUSES

(30) Priorität: 06.02.1989 DE 3903753
(43) Veröffentlichungstag der Anmeldung: 30.01.1991
(73) Patentinhaber: SCHERING AKTIENGESELLSCHAFT, 13342 Berlin (DE)
(72) Erfinder: MICHEL, Heinrich, D-1000 Berlin 20 (DE); GÖRITZ, Detlef, D-1000 Berlin 20 (DE); RÖSSLING, Georg, D-1000 Berlin 28 (DE); TACK, Johannes-Wilhelm, D-1000 Berlin 20 (DE)
(86) Internationale Anmeldenummer: DE9000028
(87) Internationale Veröffentlichungsnummer: WO9008534

## Beschreibung

Die Erfindung betrifft ein Verfahren zur Herstellung klarer, wässriger Mischmicelllösungen, enthaltend aus Lipoiden und Salzen von Gallensäuren gebildete Mischmicellen, in denen gewünschtenfalls in Wasser schwer lösliche oder unlösliche Wirkstoffe solubilisiert sind.

Verfahren zur Herstellung derartiger Mischmicellösungen sind beispielsweise aus der deutschen Patentschrift 27 30 570 vorbekannt.

Bei den vorbekannten Verfahren werden die Mischmicellösungen hergestellt, indem man die Lipoide, die Salze der Gallensäuren und gegebenenfalls die in Wasser schwer löslichen oder unlöslichen Wirkstoffe in einem organischen Lösungsmittel (z.B. Ethanol) löst, und die Lösungen einengt, so daß sich an den Gefäßwänden ein Lipidfilm bildet, der mittels wässrigen Lösungen abgelöst wird. (Biochemistry 19, 1980, 602 ff und 615 ff; Naturforsch. 32c, 1977, 748 ff).

Dieses Verfahren ist aber recht aufwendig und nur mit einem erheblichen apparativen Aufwand in einen technisch nutzbaren Maßstab zu übertragen.

Neben diesem bevorzugten Verfahren ist beispielsweise aus Beispiel 3 der bereits erwähnten Patentschrift 27 30 570 und aus der Europäischen Patentanmeldung 0280887 ein Verfahren bekannt, bei dem man derartige Mischmicellösungen durch Mischen der Komponenten und Rühren der Mischung herstellt.

Dieses Verfahren hat aber nicht nur den Nachteil, daß es mehrere Tage dauert, sondern man erkennt bei der Nacharbeitung dieses Beispiels - ohne Wirkstoff - daß man auf diese Weise nur stark getrübte Lösungen erhält, die Mischmicellen mit einem mittleren Durchmesser von ca. 340 nm enthalten.

Klare Lösungen mit Mischmicellen von einem mittleren Durchmesser von ca. 10 nm sind auf diese Weise nicht zu erhalten.

Es wurde nun gefunden, daß man in einfacher Weise und kurzer Zeit derartig klare wässrige Lösungen von Mischmicellen mittels eines Verfahrens herstellen kann, welches dadurch gekennzeichnet ist, daß man die freien Gallensäuren bei einer Temperatur von 40° C bis 100° C in einer gegebenenfalls isotonisierende Zusatze und/oder wasserlösliche Wirkstoffe enthaltenden wässrigen Lösung suspendiert, die Lipoide bei einer Temperatur von 40° C bis 100° C in dieser Suspension dispergiert und die erhaltene Dispersion bei einer Temperatur von 0° C bis 100° C mit Basen neutralisiert, mit der Maßgabe, daß man gegebenenfalls die in Wasser schwer löslichen oder unlöslichen Wirkstoffe gemeinsam mit den Lipoiden dispergiert oder in der diese Wirkstoffe nicht enthaltenden Mischmicellösung solubilisiert.

Das erfindungsgemäße Verfahren kann unter Verwendung der gleichen Gallensäuren durchgeführt werden wie die vorbekannten Verfahren. Geeignete Gallensäuren sind 5β-Cholan-24-säure-Derivate der allgemeinen Formel 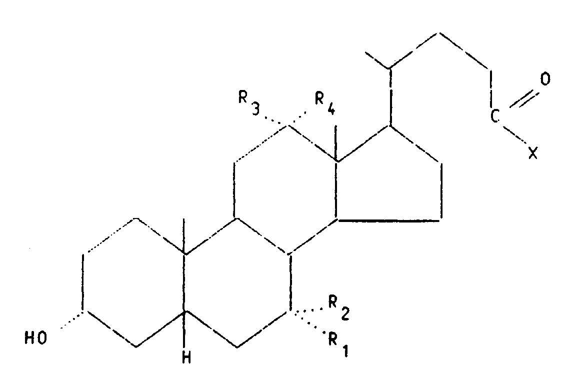
worin
R₁ und R₂ sowie R₃ und R₄ gemeinsam eine Oxogruppe, zwei Wasserstoffatome oder ein Wasserstoffatome und eine Hydroxygruppe bedeuten und
X eine Hydroxygruppe oder eine Gruppierung der Formel -NH-CH₂-CO₂H oder -NH-(CH₂)₂-SO₃H darstellt.

Als geeignete Gallensäuren seien beispielsweise genannt: Die Cholsaure, die Glycocholsäure, die Taurocholsäure, die Deoxycholsäure, die Glycodeoxycholsäure, die Taurodeoxycholsäure, die Chenodeoxycholsäure, die Glycochenodeoxycholsäure und die Taurochenodeoxycholsäure.

Zur Herstellung der wässrigen Mischmicellösung werden vorzugsweise 1 bis 30 g und insbesondere 2 g bis 15 g Gallensäure pro 100 g der gegebenenfalls isotonisierende Zusätze und wasserlösliche Wirkstoffe enthaltenden wässrigen Lösung eingesetzt.

Zur Herstellung der wassrigen Mischmicellösungen können bei dem erfindungsgemäßen Verfahren die gleichen Lipoide verwendet werden, wie bei den vorbekannten Verfahren.

Geeignete Lipoide sind beispielsweise. Monoglyceride, Sulfatide, und insbesondere Phospholipide, wie die Sphingomyeline, die Plasmalogene, die Phosphatidylcholine, die Phosphatidylethanolamine, die Phosphatidylserine, die Phosphatidylinosite und die Cardiolipine auch auch Gemische dieser Lipoide (Dr. Otto-Albert Neumüller: Römpps Chemie-Lexikon; Franck'sche Verlagshandlung, Stuttgart(DE) 2665, 3159, 3920 und 4045).

Zur Herstellung der wässrigen Mischmicellösungen werden vorzugsweise 3 bis 40 % und insbesondere 5 bis 20 % Lipoid pro 100 g der gegebenenfalls isotonisierende Zusätze und/oder wasserlösliche Wirkstoffe enthaltenden wässrigen Lösung verwendet. Das Gewichtsverhältnis zwischen Lipoid und Gallensäure beträgt vorzugsweise 0,1:1 bis 2:1 und insbesondere 0,8:1 bis 2:1.

Als Basen eignen sich zur Herstellung der wässrigen Mischmicellenlösungen nach dem erfindungsgemäßen Verfahren einerseits Alkalihydroxide, wie Lithiumhydroxid, Kaliumhydroxid und insbesondere auch Natriumhydroxid und andererseits organische Stickstoffbasen, sofern sie physiologisch unbedenkliche Salze bilden. Solche Stickstoffbasen sind beispielsweise Ammoniak, primäre, sekundäre oder tertiäre Amine, Ethanolamin, Diethanolamin, Piperazin, Morpholin, Lysin, Ornithin, Arginin, N,N-Dimethylglucamin, das Cholin und insbesondere das N-Methylglucamin und das Trishydroxymethylaminomethan.

Geeignete in Wasser schwer losliche oder unlösliche Wirkstoffe sind vorzugsweise solche, deren Löslichkeit in Wasser bei Raumtemperatur 2 % nicht übersteigt. Solche Wirkstoffe sind beispielsweise Pflanzenschutzmittel, wie schwer losliche Insektizide oder Herbizide und insbesondere schwer lösliche pharmazeutische Wirkstoffe.

In Wasser schwer lösliche oder unlösliche pharmazeutische Wirkstoffe folgender Wirkstoffgruppen eignen sich beispielsweise zur Herstellung der erfindungsgemäßen Arzneimittel:
Gestagen wirksame Steroidhormone wie beispielsweise das 13-Ethyl-17β-hydroxy-18,19-dinor-17α-pregn-4-en-20yl-3-on (=Levonorgestrel), das 13-Ethyl-17β-hydroxy-18,19-dinor-17α-pregna-4,15-dien-20yn-3-on (=Gestoden) oder das 13-Ethyl-17β-hydroxy-11-methylen-18,19-dinor-17α-pregn-4-en-20yn (Desorgestrel), Estrogen wirksame Steroidhormone wie 3-Hydroxy-1,3,5(10)-estratrien-17-on [=Ostron) oder 1,9-Nor-17α-pregna-1,3,5(10)-trien--20yn-3,17β-diol (Ethinylöstradiol).

Androgen wirksame Steroidhormone wie 17β-Hydroxy-4-androsten-3-on (=Testosteron) und dessen Ester oder 17β-Hydroxy-1α-methyl-5α-androsten-3-on (=Mesterolon).

Antiandrogen wirksame Steroidhormone wie das 17α-Acetoxy-6-chlor-1β,2β-dihydro-3 H-cyclopropa[1,2]-pregna-1,4,6-trien-3,20-dion (Cypoteronacetat).

Kortikoide wie das 11β,17α,21-Trihydroxy-4-pregnen-3,20-dion (=Hydrocortison), das 11β,17α,21-Trihydroxy-1,4-pregnadien-3,20-dion (=Prednisolon), das 11β,17α-21-Trihydroxy-6α-methyl-1,4-pregnatrien-3,20-dion (=Methylprednisolon) und das 6α-Fluor-11β,21-dihydroxy-16α-methyl-1,4-pregnadien-3,20-dion (=Difluocortolon).

Ergoline wie der 3-(9,10-Dihydro-6-methyl-8α-ergolinyl)-1,1-diethylharnstoff (=Ergolin), der 3-(2-Brom-9,10-dihydro-6-methyl-8α-ergolinyl)-1,1-diethylharnstoff (=Bromergolin) oder der 3-(6-Methyl-8α-ergolinyl)-1,1-diethylharnstoff (=Tergurid).

Antihypertonika wie das 7α-Acetylthio-17α-hydroxy-3-oxo-4-pregnen-21-carbonsäure-γ-lacton (=Spironolacton) oder das 7α-Acetylthio-15β,16β-methylen-3-oxo-17α-pregna-1,4-dien-21,17-carbolacton (=Mespirenon).

Antikoagulantia wie die 5-[Hexahydro-5-hydroxy-4-(3-hydroxy-4-methyl-1octen-6-ynyl)-2(1H)-pentalenyliden)]-pentansaure (=Iloprost).

Psychopharmaka wie das 4-(3-Cyclopentyloxy-4-methoxy-phenyl-2-pyrrolidon [=Rolipram) und das 7-Chlor-1,3-dihydro-1-methyl-5-phenyl-2H-1,4-benzodiazepin-2-on (=Diazepam).

Carotinoide wie das α-Carotin und das β-Carotin.

Fettlosliche Vitamine, wie Vitamine der Vitamin A-, Vitamin D-, Vitamin E- und Vitamin K-Gruppe.

Eine besonders bevorzugte Gruppe sind β-Carboline, wie sie beispielsweise in den Europäischen Patentanmeldungen 234,173 und 239,667 beschrieben sind. Als β-Carboline seien beispielsweise genannte der 6-Benzoyloxy-4-methoxymethyl-β-carbolin-3-carbonsäure-isopropylester (=Becarnil), der 5-(4-Chlorphenoxy)4-methoxymethyl-β-carbolin-3-carbonsäure-isopropylester (=Cl-PHOCIP), der 5-Isopropoxy-4-methyl-β-carbolin-3-carbonsäure-ethylester (=IPMCE) und das 5-(4-Chlorphenoxy)-3-(3-ethyl-1,2,4-oxadiazol-5-yl)-4-methoxymethyl-β-carbolin (=Phoco). Die optimale Wirkstoffkonzentration in den Mischmicellösungen ist naturgemäß von der Struktur des Wirkstoffes abhängig und muß mittels der geläufigen Vorversuche ermittelt werden, In der Regel beträgt die Konzentration an β-Carbolinen 1µg bis 40 mg - vorzugsweise 100 µg bis 10 mg Wirkstoff pro ml Mischmicellösung. Zur Herstellung der Mischmicellen selbst verwendet man vorzugsweise Glykocholsäure und Phospholipide.

Die nach dem erfindungsgemäßen Verfahren hergestellten wässrigen Mischmicelllösungen können gewünschtenfalls isotonische Zusätze enthalten, um deren osmotischen Druck zu erhöhen. Geeignete Zusätze sind beispielsweise anorganische oder organische Salze oder Puffersubstanzen, wie Natriumchlorid, Phosphat-Puffer, Citrat-Puffer, Glycin-Puffer, Citrat-Phosphat-Puffer, Maleat-Puffer, etc. Mono- oder Disaccharide, wie Glucose, Lactose, Saccharose, Zuckeralkohole, wie Mannit, Sorbit, Xylit oder Glycerin oder wasserlösliche Polymere, wie Dextran oder Polyethylenglykol.

Diese isotonisierenden Substanzen werden üblicherweise in solchen Konzentrationen zugesetzt, daß die entstehende wässrige Mischmicellösung einen osmotischen Druck von 5 - 1000 mosm - bei Injektionslösungen optimalerweise 300 mosm - aufweist.

Ferner können die wässrigen Mischmicellösungen noch zusätzliche wasserlösliche Wirkstoffe enthalten, um Kombinationspräparate herzustellen. Beispiele solcher Kombinationspräparate sind Mischungen aus wasserlöslichen und fettlöslichen Vitaminen oder Präparate die neben Kortikoiden noch wasserlösliche Antibiotika enthalten.

Die Herstellung der wasserlöslichen Mischmicellösungen erfolgt - abgesehen von den im Patentanspruch 1 aufgeführten Bedingungen mittels konventioneller Methoden, indem man die Mischungen unter kräftigem Rühren auf die im Patentanspruch 1 aufgeführten Temperaturen erhitzt.

Da die Lipoide und auch einige Wirkstoffe oxidationsempfindlich sind, wird das Verfahren zweckmäßigerweise unter einer Inertgasatmosphäre, wie Stickstoff oder Argon durchgeführt und die erhaltenen wässrigen Mischmicellösungen durch Zugabe von Antioxidantien, wie Natriumascorbat, Tocopherol oder Natriumhydrogensulfit stabilisiert.

Das erfindungsgemäße Verfahren kann in der Regel innerhalb weniger Minuten durchgeführt werden, es hat den Vorteil, daß Mischmicellösungen entstehen, die frei von organischen Lösungsmittelresten sind. Zudem kann das erfindungsgemäße Verfahren mit konventioneller Misch- und Dispersionstechniken (z. Beispiel nach dem Rotor-Stator-Prinzip) durchgeführt werden, wie sie in der pharmazeutischen Technologie üblich sind (siehe The Theory and Practice of Industrial Pharmacy, L. Lachman, H.A. Lieberman, J.L. Kanig, Philadelphia (1970), p. 481 oder Pharmazeutische Technologie, K.H. Bauer, K.H. Frömming und C. Führer, Stuttgart/New York (1986) p. 103)), wodurch eine gute Voraussetzung für eine reproduzierbare Prozeßführung, insbesondere unter GMP-Bedingungen (Good Manufacturing Practices) gegeben ist.

Nach erfolgter Herstellung kann die erhaltene wässrige Mischmicellösung sterilfiltriert und/oder bei 100° C bis 140° C hitzesterilisiert werden.

Die nachfolgenden Ausführungsbeispiele dienen zur näheren Erläuterung des erfindungsgemäßen Verfahrens.

### Beispiel 1

700 ml 0,02 n Acetatpuffer von pH 6,0 werden in einem beheizbaren Rühr- und Homogenisiergerät unter Einleiten von Argon auf 80° C erwärmt. Dann setzt man unter Rühren 54,2 g Glykocholsäure zu und rührt noch bis eine homogene Suspension entsteht (ca. 5 Minuten) Dann setzt man der Suspension 90 g Phosphatidylcholin (Sojalecitin) und 10 g Becarnil =6-Benzoyloxy-4-methoxymethyl-β-carbolin-3-carbonsäure-isopropyester zu und rührt bei 80° C bis die Dispersion homogen ist (ca. 10 Minuten), Danach versetzt man mit 20 %iger wässriger Natriumhydroxidlösung bis ein pH-Wert von 6,0 erreicht ist und erhält sofort eine klare Lösung, die nach dem Abkühlen mit Wasser aufgefüllt wird, so daß ein Endvolumen von 1000 ml erreicht ist. Die so erhaltene wässrige Mischmicelllösung wird sterilfiltriert.

### Beispiel 2

700 ml 0,015 m Phosphatpuffer von pH 7,0 werden in einem beheizbaren Rühr- und Homogenisiergerät unter Einleiten von Argon auf 80° C erwärmt. Dann setzt man unter Rühren 47,6 g Cholsäure zu und rührt noch bis eine homogene Suspension entsteht (ca. 5 Minuten) Dann setzt man der Suspension 90 g Phosphatidylcholin (Sojalecitin) zu und rührt bei 80° C bis die Dispersion homogen ist (ca. 10 Minuten). Danach versetzt man mit 20 %iger wässriger Natriumhydroxidlösung bis ein pH-Wert von 7,0 erreicht ist und erhält sofort eine klare Lösung. In diese Lösung werden 10 g Becarnil =6-Benzoyloxy-4-methoxymethyl-β-carbolin-3-carbonsäure-isopropylester eingerührt, die sich rasch auflösen. Die erhaltene Lösung wird nach dem Abkühlen mit Wasser aufgefüllt, so daß ein Endvolumen von 1000 ml erreicht ist. Die so erhaltene wässrige Mischmicellösung wird bei 121° C sterilisiert.

### Beispiel 3

700 ml 0,02 N Acetatpuffer von pH 6.0 werden in einem beheizbaren Rühr- und Homogenisiergerät unter Einleiten von Argon auf 80° C erwärmt. Dann setzt man unter Rühren 54,2 Glykocholsäure zu und rührt noch bis eine homogene Suspension entsteht (ca. 5 Minuten). Dann setzt man der Suspension 90 g Phophatidylcholin (Phospholipon 100 L, Fa. Nattermann) und 1,1 g Cl-Phocip =6-Benzoyloxy-4-methoxymethyl-β-carbolin-3-carbonsäure-isopropylester zu und rührt bei dieser Temperatur bis die Dispersion homogen ist. Nach dem Abkühlen auf 40 - 50° C wird mit 20 %iger wässriger Natriumhydroxidlösung neutralisiert, wobei eine klare Lösung mit einem pH-Wert von 6,0 entsteht. Nach dem Auffüllen auf das Endvolumen auf 1000 ml wird die wässrige Mischmicellenlösung sterilfiltriert und kann gegebenenfalls bei 121° C sterilisiert werden.

### Beispiel 4

700 ml Wasser für Injektionszwecke werden in einem beheizbaren Rühr- und Homogenisiergerät unter Einleiten von Argon auf 80° C erwärmt. Dann setzt man unter Rühren 54,2 g Glykocholsäure zu und rührt noch bis eine homogene Suspension entsteht (ca. 5 Minuten). Dann setzt man der Suspension 90 g Phophatidylcholin und 2 g IPMCE =5-Isopropoxy-4-methyl-β-carbolin-3-carbonsäureethylester zu und rührt bei dieser Temperatur bis die Dispersion homogen ist (ca. 10 - 15 Minuten). Danach versetzt man mit 20 %iger wässriger Natriumhydroxidlösung bis ein pH-Wert von 6,0-6,5 erreicht ist und erhält eine klare Lösung die nach dem Abkühlen mit Wasser auf 1000 ml aufgefüllt wird. Die so erhaltene wässrige Mischmicellenlösung wird sterilfiltriert.

### Beispiel 5

700 ml Wasser für Injektionszwecke werden in einem beheizbaren Rühr- und Homogenisjergerät unter Einleiten von Argon auf 80° C erwärmt. Dann setzt man unter Rühren 54,2 g Glykocholsäure zu und rührt noch bis eine homogene Suspension entsteht (ca. 5 Minuten). Dann setzt man der Suspension 90 g Phophatidylcholin und 1 g Phoco =5-(4-Chlorphenoxy)-3-(3-ethyl-1,2,4-oxadiazol-5-yl-4-methoxymethyl-β-carbolin zu und rührt bei dieser Temperatur bis die Dispersion homogen ist (ca. 10 - 15 Minuten). Danach versetzt man mit 20 %iger wässriger Natriumhydroxidlösung bis ein pH-Wert von 6,0-6,5 erreicht ist und erhält eine klare Lösung die nach dem Abkühlen mit Wasser auf 1000 ml aufgefüllt wird. Die so erhaltene wässrige Mischmicellenlösung wird sterilfiltriert.

## Patentansprüche

1. Verfahren zur Herstellung klarer wässriger Mischmicellösungen, enthaltend aus Lipoiden und Salzen von Gallensäuren gebildete Mischmicellen, in denen gewünschtenfalls in Wasser schwer lösliche oder unlösliche Wirkstoffe solubilisiert sind, dadurch gekennzeichnet, daß man die freien Gallensäuren bei einer Temperatur von 40° C bis 100° C in einer gegebenenfalls isotonisierende Zusätze und/oder wasserlösliche Wirkstoffe enthaltenden wässrigen Lösung suspendiert, die Lipoide bei einer Temperatur von 40° C bis 100° C in dieser Suspension dispergiert und die erhaltene Dispersion bei einer Temperatur von 0° C bis 100° C mit Basen neutralisiert, mit der Maßgabe, daß man gegebenenfalls die in Wasser schwer löslichen oder unlöslichen Wirkstoffe gemeinsam mit den Lipoiden dispergiert oder in der diese Wirkstoffe nicht enthaltenden Mischmicellösung solubilisiert.

2. Verfahren zur Herstellung klarer wässriger Mischmicellösungen gemäß Patentanspruch 1, dadurch gekennzeichnet, daß man als Gallensäure ein 5β-Cholan-24-säure-Derivat der allgemeinen Formel 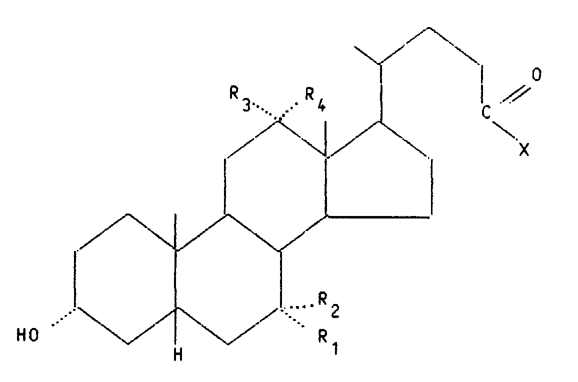 worin
R₁ und R₂ sowie R₃ und R₄ gemeinsam eine Oxogruppe, zwei Wasserstoffatome oder ein Wasserstoffatom und eine Hydroxygruppe bedeuten und
X eine Hydroxygruppe oder eine Gruppierung der Formel -NH-CH₂-CO₂H oder -NH-(CH₂)₂-SO₃H darstellt, verwendet.

3. Verfahren zur Herstellung klarer, wässriger Mischmicellösungen gemäß Patentanspruch 1 und 2, dadurch gekennzeichnet, daß man als Lipoide Phospholipide verwendet.

4. Verfahren zur Herstellung klarer, wässriger Mischmicellösungen gemäß Patentanspruch 1 bis 3, dadurch gekennzeichnet, daß man als in Wasser schwer lösliche oder unlösliche Wirkstoffe pharmazeutische Wirkstoffe verwendet.

5. Verfahren zur Herstellung klarer, wässriger Mischmicellösungen gemäß Patentanspruch 4, dadurch gekennzeichnet, daß man als pharmazeutische Wirkstoffe β-Carboline verwendet.

6. Verwendung von gemäß Patentanspruch 4 und 5 hergestellten klaren, wässrigen Mischmicellösungen zur Herstellung von Injektionslösungen.

7. Klare, wässrige Mischmicellösungen enthaltend β-Carboline.

8. Klare, wässrige Mischmicellösungen enthaltend 6-Benzoyloxy-4-methoxymethyl-β-carbolin-3-carbonsäure-isopropylester.

9. Klare, wässrige Mischmicellösungen enthaltend 5-(4-Chlorphenoxy)-4-methoxymethyl-β-3-carbonsäure-isopropylester.

10. Klare, wässrige Mischmicellösungen enthaltend 5-Isopropoxy-4-methyl-β-carbolin-3-carbonsäure-ethylester.

11. Klare, wässrige Mischmicellösungen enthaltend 5-(4-Chlorphenoxy)-3-(3-ethyl-1,2,4-oxadiazol-5-yl)-4-methoxymethyl-β-carbolin.

## Claims

1. Process for the preparation of clear aqueous mixed micelle solutions, containing mixed micelles formed from lipids and salts of bile acids, in which, if desired, active ingredients that are insoluble or sparingly soluble in water are solubilised, characterised in that the free bile acids are suspended at a temperature of from 40°C to 100°C in an aqueous solution optionally containing isotonising additives and/or water-soluble active ingredients, the lipids are dispersed in that suspension at a temperature of from 40°C to 100°C and the resulting solution is neutralised with bases at a temperature of from 0°C to 100°C, with the proviso that, where appropriate, the active ingredients insoluble or sparingly soluble in water are dispersed together with the lipids or solubilised in the mixed micelle solution not containing those active ingredients.

2. Process for the preparation of clear aqueous mixed micelle solutions according to patent claim 1, characterised in that there is used as bile acid a 5β-cholanic-24 acid derivative of the general formula 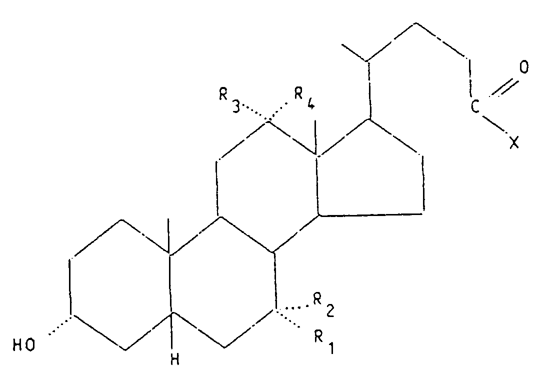 wherein
R₁ and R₂ and also R₃ and R₄ together represent an oxo group, two hydrogen atoms, or a hydrogen atom and a hydroxy group, and
X represents a hydroxy group or a grouping of the formula -NH-CH₂-CO₂H or -NH-(CH₂)₂-SO₃H.

3. Process for the preparation of clear aqueous mixed micelle solutions according to patent claim 1 and 2, characterised in that the lipids used are phospholipids.

4. Process for the preparation of clear aqueous mixed micelle solutions according to any one of patent claims 1 to 3, characterised in that pharmaceutically active ingredients are used as active ingredients that are insoluble or sparingly soluble in water.

5. Process for the preparation of clear, aqueous mixed micelle solutions according to patent claim 4, characterised in that β-carbolines are used as pharmaceutically active ingredients.

6. Use of clear aqueous mixed micelle solutions prepared in accordance with patent claim 4 and 5 for the preparation of injection solutions.

7. Clear aqueous mixed micelle solutions containing β-carbolines.

8. Clear aqueous mixed micelle solutions containing 6-benzoyloxy-4-methoxymethyl-β-carboline-3-carboxylic acid isopropyl ester.

9. Clear aqueous mixed micelle solutions containing 5-(4-chlorophenoxy)-4-methoxymethyl-β-3-carboxylic acid isopropyl ester.

10. Clear aqueous mixed micelle solutions containing 5-isopropoxy-4-methyl-β-carboline-3-carboxylic acid ethyl ester.

11. Clear aqueous mixed micelle solutions containing 5-(4-chlorophenoxy)-3-(3-ethyl-1,2,4-oxadiazol-5-yl)-4-methoxymethyl-β-carboline.

## Revendications

1. Procédé de préparation de solutions aqueuses claires de micelles mixtes contenant des micelles mixtes formées à partir de lipoïdes et de sels d'acides biliaires et dans lesquelles éventuellement peuvent être solubilisées si l'on veut des matières actives peu solubles ou insolubles dans l'eau, procédé caractérisé en ce que l'on met les acides biliaires libres, à une température de 40 à 100°C, dans une solution aqueuse pouvant contenir éventuellement des additifs isotonisants et/ou des matières actives hydrosolubles, puis on disperse les lipoïdes dans cette suspension à une température de 40 à 100°C et on neutralise la dispersion obtenue avec une base entre 0 et 100°C, avec la condition de disperser les matières actives éventuellement ajoutées, peu solubles ou insolubles dans l'eau, en même temps que les lipoïdes, ou bien de les solubiliser dans la solution de micelles mixtes ne contenant pas ces matières actives.

2. Procédé selon la revendication 1 caractérisé en ce que l'acide biliaire est un dérivé de l'acide 5β-cholanique-24 de formule générale 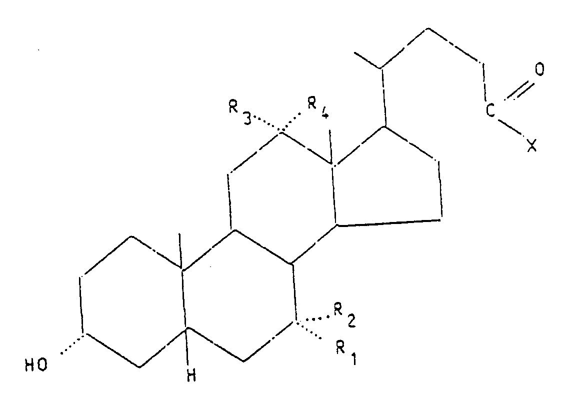 dans laquelle
R₁ et R₂, ainsi que R₃ et R₄, respectivement, forment ensemble un groupe oxo ou bien représentent deux atomes d'hydrogène ou un atome d'hydrogène et un groupe hydroxylique, et
X est un groupe hydroxylique ou un groupe -NH-CH₂-CO₂H ou -NH-(CH₂)₂-SO₃H.

3. Procédé selon la revendication 1 ou 2, caractérisé en que les lipoïdes sont des phospholipides.

4. Procédé selon les revendications 1 à 3, caractérisé en ce que les matières actives peu solubles ou insolubles dans l'eau sont des produits pharmaceutiques.

5. Procédé selon la revendication 4, caractérisé en ce que les produits pharmaceutiques sont des β-carbolines.

6. L'emploi de solutions aqueuses claires de micelles mixtes obtenues selon les revendications 4 et 5 pour en préparer des solutions injectables.

7. Solutions aqueuses claires de micelles mixtes contenant des β-carbolines.

8. Solutions aqueuses claires de micelles mixtes contenant du 6-benzoyloxy-4-méthoxyméthyl-β-carboline-3-carboxylate d'isopropyl.

9. Solutions aqueuses claires de micelles mixtes contenant du 5-(4-chlorophénoxy)-4-méthoxyméthyl-β-carboline-3-carboxylate d'isopropyle.

10. Solutions aqueuses claires de micelles mixtes contenant du 5-isopropoxy-4-méthyl-β-carboline-3-carboxylate d'éthyle.

11. Solutions aqueuses claires de micelles mixtes contenant de la 5-(4-chlorophénoxy)-3-(3-éthyl-1,2,4-oxadiazol-5-yl)-4-méthoxyméthyl-β-carboline.
